# EUROPEAN PATENT APPLICATION

(11) **EP 1 800 596 A1**
(43) Date of publication of application: **27.06.2007**
(21) Application number: 06024618.8
(22) Date of filing: 28.11.2006
(51) Int. Cl.: A61B 1/12, A61M 25/10

(54) **Overtube for endoscope and endoscope system**

(30) Priority: 21.12.2005 JP 2005368362
(71) Applicant: OLYMPUS MEDICAL SYSTEMS CORP., Shibuya-ku Tokyo 151-0072 (JP)
(72) Inventor: Matsui, Raifu Olympus I. P. Services Co.,LTD., Hachioji-shi Tokyo 192-8512 (JP); Matsuura, Nobuyuki Olympus I. P. Services Co.,LTD., Hachioji-shi Tokyo 192-8512 (JP)
(74) Representative: von Hellfeld, Axel

(57) **Abstract**

An overtube (14) for an endoscope (12) includes a tube body (72), a balloon (74), a first-communication-path (86), a second-communication-path (96), a first-mouthpiece (82), a second-mouthpiece (92), a first-connection-section (84) which is provided on the first-mouthpiece, and a second-connection-section (94) which is provided on the second-mouthpiece. The balloon is dilatably/deflatably provided on the tube body. The first-communication-path extends from a distal-end-portion toward a proximal-end-portion of the tube body in a state in which the first-communication-path communicates with the balloon. The second-communication-path extends from a hollow-portion toward the proximal-end-portion of the tube body. The first-mouthpiece projects from the tube body and includes a path communicating with the first-communication-path. The second-mouthpiece projects from the tube body and includes a path communicating with the second-communication-path. The first-connection-section is connected to a first-feed-portion (106) of a fluid-feed-mechanism and is prevented from being connected to a second-feed-portion (116) of the fluid-feed-mechanism. The second-connection-section is connected to the second-feed-portion and is prevented from being connected to the first-feed-portion.

## Description

The present invention relates to an overtube for an endoscope, which is used for an endoscope that is inserted into a body cavity in a peroral or per-anum manner in order to perform observation, surgery, etc., within the body cavity, and also relates to an endoscope system.

Jpn. Pat. Appln. KOKAI Publication No. 2004-337288, for instance, discloses an overtube for use with an endoscope. This overtube has two mouthpieces. One of the mouthpieces is provided in order to dilate and deflate a balloon, and the other mouthpiece is provided in order to feed, e.g., a lubricant (physiological saline) into the overtube. The mouthpieces of the overtube disclosed in the KOKAI Publication No. 2004-337288 are formed to have different shapes and sizes, thereby to prevent erroneous connection.

In the overtube disclosed in the KOKAI Publication No. 2004-337288, depending on the degree of differences in shape and size of the two mouthpieces, the connection sections may be damaged if the mouthpieces are forcibly connected. If the difference in shape of the mouthpieces is too small, it is difficult, in some cases, to distinguish between a connectable and a non-connectable member.

The present invention has been made in consideration of the above-described problem, and the object of the invention is to provide an overtube for an endoscope and an endoscope system, which can easily determine non-connectability between erroneously combined connection members, and can easily determine mutually connectable members and can connect these members.

According to one aspect of the present invention, there is provided an overtube for an endoscope which comprises a tube body, a balloon, a first communication path, a second communication path, a first mouthpiece, a second mouthpiece, a first connection section which is provided on the first mouthpiece, and a second connection section which is provided on the second mouthpiece. The tube body includes a hollow portion in which an insertion section of the endoscope is inserted. The balloon is dilatably and deflatably provided on an outer peripheral surface of the tube body on a distal end side of the tube body. The first communication path extends from a distal end portion of the tube body toward a proximal end portion of the tube body in a state in which the first communication path communicates with the balloon. The second communication path extends from the hollow portion toward the proximal end portion of the tube body in a state in which the second communication path communicates with the hollow portion. The first mouthpiece projects from the outer peripheral surface of the tube body and includes a path communicating with the first communication path. The second mouthpiece projects from the outer peripheral surface of the tube body and includes a path communicating with the second communication path. The first connection section is connected to a first feed portion of a fluid feed mechanism and is prevented from being connected to a second feed portion of the fluid feed mechanism. The second connection section is connected to the second feed portion of the fluid feed mechanism and is prevented from being connected to the first feed portion of the fluid feed mechanism.

According to another aspect of the present invention, there is provided an endoscope system comprising an endoscope and an overtube. The endoscope includes an elongated insertion section having a bendable bend portion at a distal end side thereof, and an operation section which is provided at a proximal end portion of the insertion section and is capable of performing an operation of bending the bend portion. The overtube includes a tube body, a balloon, a first communication path, a second communication path, a first connection section, and a second connection section. The tube body includes a hollow portion in which the insertion section is detachably inserted such that the tube body covers a part of the insertion section. The balloon is provided on an outer peripheral surface of the tube body on a distal end side of the tube body. The first communication path has one end communicating with the balloon and the other end extending toward the proximal end portion of the tube body along the outer peripheral surface of the tube body. The second communication path has one end communicating with the hollow portion of the tube body and the other end extending toward the proximal end portion of the tube body. The first connection section is provided on an outside of the tube body, includes a first mouthpiece communicating with the other end of the first communication path, is connectable to a first fluid feed mechanism which feeds and drains a fluid to and from the first communication path, and is prevented from being connected to a second fluid feed mechanism which feeds and drains a fluid to and from the second communication path. The second connection section is provided on the outside of the tube body, formed in a shape different from a shape of the first connection section, includes a second mouthpiece communicating with the other end of the second communication path, is connectable to the second fluid feed mechanism which feeds and drains a fluid to and from the second communication path, and is prevented from being connected to the first fluid feed mechanism.

The invention can be more fully understood from the following detailed description when taken in conjunction with the accompanying drawings, in which:
FIG. 1 schematically shows an endoscope system including an endoscope and an overtube for the endoscope according to a first embodiment of the present invention;
FIG. 2A is a schematic, partial longitudinal sectional view of the overtube for the endoscope in the endoscope system according to the first embodiment;
FIG. 2B schematically shows the overtube in a case where the overtube for the endoscope in the endoscope system according to the first embodiment is observed in a direction of arrow 2B in FIG. 2A;
FIG. 3A is a schematic longitudinal sectional view of a proximal-end-side part of the overtube for the endoscope in the endoscope system according to the first embodiment;
FIG. 3B is a schematic longitudinal sectional view showing a state in which tube connection portions are connected to proximal-end-side connection sections of the overtube for the endoscope in the endoscope system according to the first embodiment;
FIG. 4A to FIG. 4C schematically show overtubes for an endoscope in an endoscope system according to a second embodiment of the invention; and
FIG. 5 is a schematic longitudinal sectional view showing a state in which an overtube is fitted over an insertion section of an endoscope of an endoscope system according to a third embodiment of the invention, and an additional balloon for an endoscope is mounted on a distal end portion of the insertion section.

Preferred embodiments of the present invention will now be described with reference to the accompanying drawings.

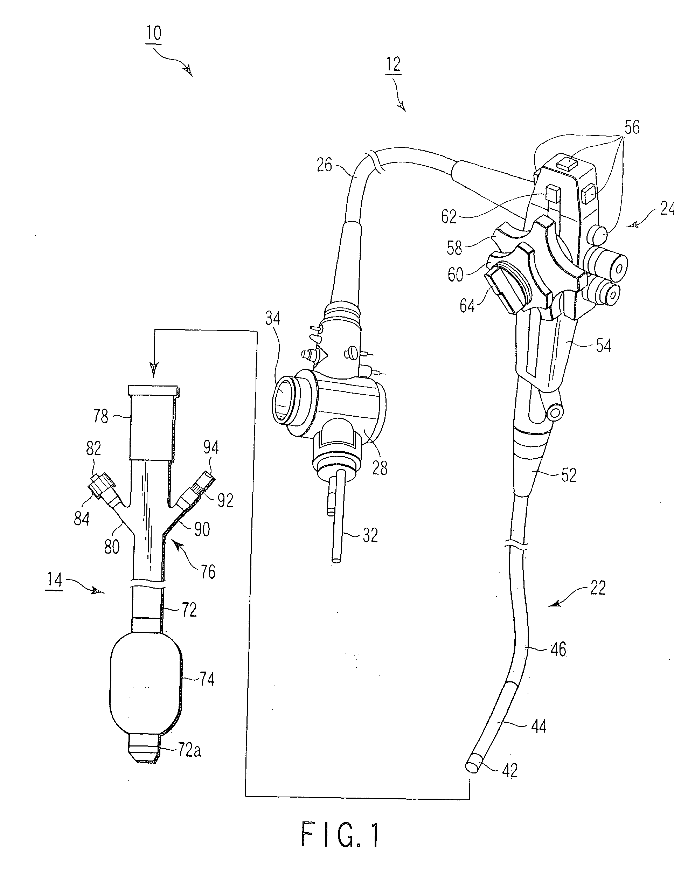

A first embodiment of the invention is described with reference to FIG. 1 to FIG. 3B.

As is shown in FIG. 1, an endoscope system 10 according to the first embodiment includes an endoscope 12 and an overtube 14.

The endoscope 12 includes an elongated insertion section 22 and an operation section 24 which is connected to a proximal end portion of the insertion section 22. One end portion of a universal cable 26, which can transmit illumination light and various signals from a light source device (not shown), extends from a proximal end portion of the operation section 24. A connector section 28 is provided at the other end portion of the universal cable 26. The connector section 28 includes a light guide connector 32 and an electrical connector 34. The light guide connector 32 is disposed on the same axis as the universal cable 26. The above-mentioned light source device is connected to the light guide connector 32. The electrical connector 34 is formed on a sidewall surface of the connector section 28. A camera cable for connection to a camera control unit (not shown) is connected to the electrical connector 34.

A monitor (not shown) is connected to the camera control unit. As a result, if an optical image of an examined part is captured by a solid-state image sensing device such as a CCD (described later), the signal relating to the optical image is processed by the camera control unit and the captured image of the examined part is displayed on the monitor.

The insertion section 22 includes a rigid distal-end structure unit 42, a bend portion 44 which is bendable up, down, right and left, and a flexible tube portion 46 which is elongated and has flexibility.

The distal-end structure unit 42 is provided at an endmost position of the insertion section 22. The distal-end structure unit 42 is equipped with an illumination optical system, an observation optical system of, e.g., the solid-state image sensing device, a forceps opening communicating with a surgical instrument insertion channel, and a nozzle for supplying air into a body cavity and water to an observation lens (these components are not shown). The surgical instrument insertion channel communicates with a surgical instrument insertion port (not shown) of the operation section 24.

A distal end portion of the bend portion 44 is connected to a proximal end portion of the distal-end structure unit 42. A distal end portion of the flexible tube portion 46 is connected to a proximal end portion of the bend portion 44. A distal end portion of the operation section 24 is connected to a proximal end portion of the insertion section 22. In other words, the distal end portion of the operation section 24 is connected to a proximal end portion of the insertion section 22.

The distal end portion of the operation section 24 is provided with a support portion 52 which supports the proximal end portion of the flexible tube portion 46. A distal end portion of the support portion 52 is tapered toward the proximal end portion of the flexible tube portion 46 of the insertion section 22. A proximal end portion of the support portion 52 is provided with a grip 54 which is a hold portion that is held by an operator. The grip 54 is provided with remote switches 56 for remote-controlling a video recording device such as a VTR (not shown), a camera control unit (not shown), etc.

Bend operation levers 58 and 60, which are rotated and operated by the operator, are provided at a proximal end portion of the grip 54. If the bend operation levers 58, 60 are operated, the above-mentioned bend portion 44 is bent in directions away from the longitudinal axis of the flexible tube portion 46, for example, up and down, and right and left. The operation lever that is designated by reference number 58 is used, for example, for bending up and down. The operation lever that is designated by reference number 60 is used, for example, for bending right and left.

Adjacent to the bend operation lever 58, a bend fixing lever 62 is provided which fixes the bend operation lever 58 at a desired position and thus fixes the bend portion 44 in the state in which the bend portion 44 has a desired degree of bend. This lever 62 is also operated when the fixation of the bend portion 44 is to be released. In short, the lever 62 is operated in the case of fixing the bend portion 44 in a desired state and in the case of releasing the fixation of the bend portion 44 and setting the bend operation lever 58 in a movable state.

Like the bend operation lever 58, the other bend operation lever 60 is provided with a bend fixing lever 64. The lever 64 is also operated when the fixation of the bend portion 44 is to be released. In short, the lever 64 is operated in the case of fixing the bend portion 44 in a desired state and in the case of releasing the fixation of the bend portion 44 and setting the bend operation lever 60 in a movable state.

In order to facilitate the insertion of the insertion section 22 of the endoscope 12 having the above-described structure, the overtube 14 for the endoscope 12 shown in FIG. 1 is used in the state in which the overtube 14 is fitted over a part of the insertion section 22.

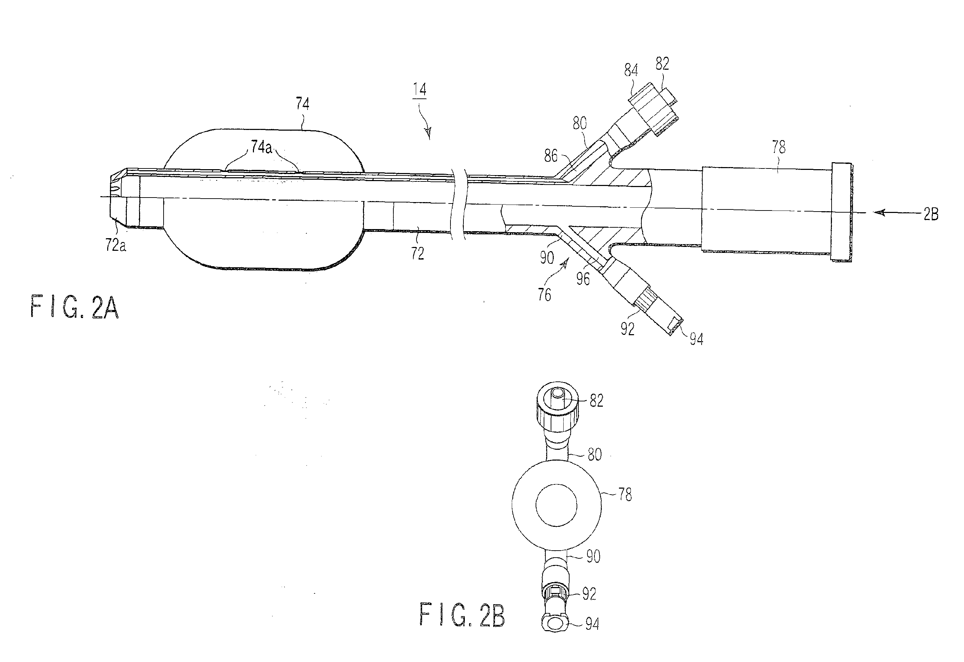

As is shown in FIG. 2A, the overtube 14 for the endoscope 12, which is detachably fitted on the insertion section 22 of the endoscope 12, includes an elongated cylindrical tube body 72, a dilatable/deflatable balloon 74, a fluid connection section 76, and a proximal-end-side hold portion 78. The tube body 72 includes a hollow portion in which the insertion section 22 of the endoscope 12 is inserted. The tube body 72 has flexibility, like the flexible tube portion 46 of the insertion section 22 of the endoscope 12. Thus, if the flexible tube portion 46 of the insertion section 22 of the endoscope 12 is bent due to force from the body wall, the tube body 72 is bent accordingly.

The balloon 74 is provided on an outer peripheral surface in the vicinity of the distal end portion of the tube body 72. A rigid distal-end tip 72a is provided at the distal end portion of the tube body 72. On the other hand, a proximal-end-side hold portion 78 is provided at a proximal end portion of the tube body 72. The proximal-end-side hold portion 78 is formed of, e.g., a rigid material so that the hold portion 78 may easily be held.

The fluid connection section 76 is projected at the proximal-end side of the tube body 72 and at the distal-end side of the proximal-end-side hold portion 78. The fluid connection section 76 includes first and second rigid portions 80 and 90. The first and second rigid portions 80 and 90 extend from proximal-end-side proper positions (proximal parts of extension) of the overtube 14 toward the proximal end portion of the overtube 14. Specifically, the first and second rigid portions 80 and 90 extend on the proximal end side of the tube body 72 in directions away from the axis of the tube body 72. The first and second rigid portions 80 and 90 are formed at mutually opposed positions with respect to the center axis of the tube body 72.

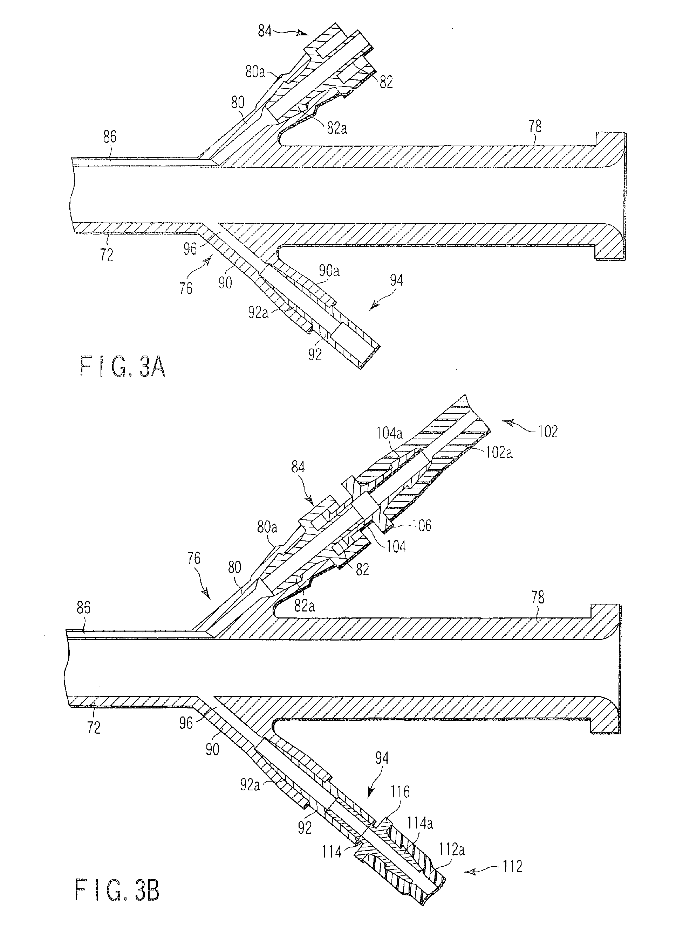

As is shown in FIG. 2A, FIG. 2B and FIG. 3A, a first mouthpiece 82 is provided at an end portion (extension end portion) of the first rigid portion 80. The first mouthpiece 82 is provided with a first connection section 84.

As shown in FIG. 2A and FIG. 3A, a first communication path 86 is formed from the distal end portion of the tube body 72 to the first rigid portion 80. The first communication path 86 is formed in the tube body 72 itself so as to extend along the axis of the tube body 72. In the vicinity of a distal end portion of the first communication path 86, a plurality of openings 74a, which communicate with the outside of the tube body 72 and are located within the balloon 74, are formed. Thus, gas may be fed from a proximal end portion of the first communication path 86, thereby to dilate the balloon 74. Needless to say, gas may be drawn to deflate the balloon 74. In the case where gas is fed/drawn, the feeding/drawing of the gas is performed by a first fluid feed mechanism (not shown).

In addition, as shown in FIG. 3A, a first mouthpiece hold portion 80a, in which the first mouthpiece 82 is mounted, is formed on the first rigid portion 80. A mouthpiece fixing portion 82a, which is formed at the distal end of the first mouthpiece 82, is held in the first mouthpiece hold portion 80a. At this time, a hollow portion of the first mouthpiece 82 communicates with the first communication path 86.

The first connection section 84 is formed at the first mouthpiece 82. The first connection section 84 is cylindrically formed in such a state that the first connection section 84 is spaced apart by a predetermined distance from the first mouthpiece 82 by a flange portion which projects radially outward from the first mouthpiece 82. A proximal end portion of the first rigid portion 80 is abutted upon the flange portion of the first connection section 84. In short, an opening is defined between the first mouthpiece 82 and the proximal end portion of the first connection section 84.

The first connection section 84 cooperates with a first tube connection portion (first feed portion) 106 which is provided at a distal end of a first connection tube 102 shown in FIG. 3B, thus communicating the first mouthpiece 82 with a first tube mouthpiece 104. An end portion (not shown) of the first connection tube 102 is connected to a first fluid feed mechanism (not shown) which feeds/draws gas to/from the balloon 74 through the first communication path 86. The first tube connection portion 106 is abutted upon a proximal end surface of the first connection section 84 by a flange portion which projects radially outward from the first tube mouthpiece 104.

The first tube mouthpiece 104 is held by a first tube mouthpiece fixing portion 104a in a first tube mouthpiece hold portion 102a which is provided at the distal end of the first connection tube 102.

The first connection section 84 of the first mouthpiece 82 of the tube body 72 has a male configuration, and the first tube mouthpiece 104 has a female configuration. Thus, the first connection section 84 of the first mouthpiece 82 of the tube body 72 is engaged with, and detachably connected to, the first tube connection section 106 of the first tube mouthpiece 104 of the first connection tube 102.

As is shown in FIG. 2A, FIG. 2B and FIG. 3A, a second mouthpiece 92 is provided at an end portion (extension end portion) of the second rigid portion 90. A second connection section 94 is provided at the second mouthpiece 92.

As shown in FIG. 2A and FIG. 3A, a second communication path 96 is formed from the tube body 72 to the second rigid portion 90. The second communication path 96 is formed along the axis of the second rigid portion 90. One end of the second communication path 96 communicates with the hollow portion of the tube body 72.

Further, as shown in FIG. 3A, a second mouthpiece hold portion 90a, in which the second mouthpiece 92 is mounted, is formed at the second rigid portion 90. A mouthpiece fixing portion 92a, which is provided at the distal end of the second mouthpiece 92, is held in the second mouthpiece hold portion 90a. At this time, a hollow portion of the second mouthpiece 92 communicates with the second communication path 96.

The second connection section 94 is formed at the second mouthpiece 92. The second connection section 94 cooperates with a second tube connection portion (second feed portion) 116 which is provided at a distal end of a second connection tube 112 shown in FIG. 3B, thereby communicating the second mouthpiece 92 with a second tube mouthpiece 114. An end portion (not shown) of the second connection tube 112 is connected to a second fluid feed mechanism (not shown) which feeds water and air into the hollow portion of the tube body 72 through the second communication path 96. The second tube connection portion 116 is abutted upon a proximal end surface of the second connection section 94 by a flange portion which projects radially outward from the second tube mouthpiece 114.

The second tube mouthpiece 114 is held by a second tube mouthpiece fixing portion 114a in a second tube mouthpiece hold portion 112a which is provided at the distal end of the second connection tube 112.

The second connection section 94 of the second mouthpiece 92 of the tube body 72 has a female configuration, and the second tube mouthpiece 114 has a male configuration. Thus, the second connection section 94 of the second mouthpiece 92 of the tube body 72 is engaged with, and detachably connected to, the second tube connection section 116 of the second tube mouthpiece 114 of the second connection tube 112.

Next, the operation of the endoscope system 10 according to the present embodiment is described.

To begin with, the insertion section 22 of the endoscope 12 is inserted in the hollow portion of the overtube 14. The first tube connection portion 106 of the first connection tube 102 is connected to the first connection section 84 of the first mouthpiece 82. The second tube connection portion 116 of the second connection tube 112 is connected to the second connection section 94 of the second mouthpiece 92. In this case, the first connection section 84 has a male configuration, and the second connection section 94 has a female configuration. The first tube connection portion 106 has a female configuration, and the second tube connection portion 116 has a male configuration. Therefore, it is possible to prevent the second tube connection portion 116 from being connected to the first connection section 84, and to prevent the first tube connection portion 106 from being connected to the second connection section 94.

The first connection section 84 and second connection section 94 are disposed at symmetrical positions with respect to the tube body 72. In addition, the proximal portion of the first connection section 84 is the first rigid portion 80, and the proximal portion of the second connection section 94 is the second rigid portion 90. Therefore, even in a dark operating theater, it is possible to prevent the second tube connection portion 116 from being connected to the first connection section 84, and to prevent the first tube connection portion 106 from being connected to the second connection section 94.

In the state in which the balloon 74 is deflated, the distal end portion of the tube body 72 of the overtube 14 and the distal end portion of the insertion section 22 of the endoscope 12 are inserted, for example, into the large intestine. In this case, the insertion section 22 is placed deep into the large intestine while the bend portion 44 is being bent in a desired direction by operating the bend operation levers 58, 60. When the insertion section 22 is to be placed deeper into the large intestine, the balloon 74 is dilated (expanded) through the first communication path 86 from the first connection tube 102. The dilation of the balloon 74 creates a space on the front side of the insertion section 22 within the large intestine (i.e., on the distal-end side of the insertion section 22). In other words, by the dilation of the balloon 74, the lumen in the large intestine is largely dilated by the outer peripheral surface of the balloon 74. In this state, a liquid, such as physiological saline, is injected into the hollow portion of the tube body 72 from the second connection tube 112 through the second communication path 96. The liquid, such as physiological saline, serves as a lubricant for decreasing friction between the inner peripheral surface of the tube body 72 and the outer peripheral surface of the insertion section 22 of the endoscope 12. The insertion section 22 of the endoscope 12 is moved forward, relative to the tube body 72 of the overtube 14.

Gas within the balloon 74 is drawn through the first communication path 86, and the balloon 74 is deflated. The tube body 72 is moved toward the distal end of the insertion section 22 along the insertion section 22.

When the insertion section 22 is to be inserted still deeper into the large intestine, the balloon 74 is dilated once again. The insertion section 22 is further inserted toward the space that is created by the balloon 74. Thereafter, the balloon 74 is deflated. If a space is needed for surgery, the balloon 74 may be kept in the dilated state.

As has been described above, the following advantageous effect can be obtained by the present embodiment.

The first and second rigid portions 80 and 90, which are provided on the tube body 72 of the overtube 14, are symmetrically disposed with respect to the center axis of the overtube 14. In addition, the first mouthpiece 82 that is provided on the first rigid portion 80 has a male configuration, and the second mouthpiece 92 that is provided on the second rigid portion 90 has a female configuration. Thus, only the female first tube mouthpiece 104 can be engaged with the first mouthpiece 82, and the male second tube mouthpiece 114 can be engaged with the second mouthpiece 92. Therefore, it is possible to prevent tubes from erroneously being connected to the first and second mouthpieces 82 and 92.

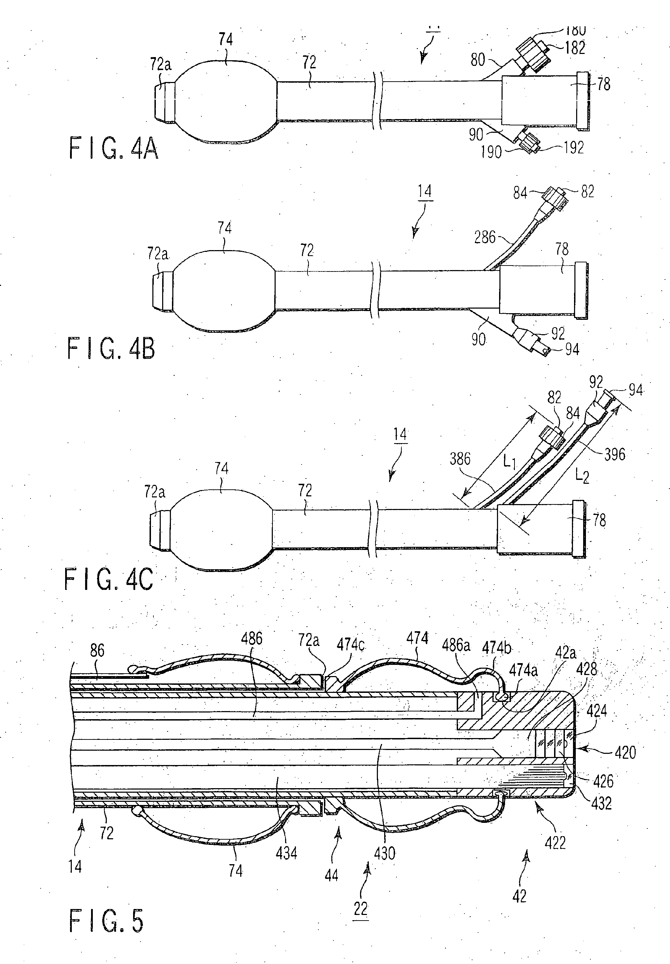

A second embodiment of the invention is described with reference to FIG. 4A to FIG. 4C. The second embodiment relates to modifications of the first embodiment. The same parts as described in the first embodiment or the parts with the same functions as described in the first embodiment are denoted by like reference numbers, and detailed description thereof is omitted.

As is shown in FIG. 4A, first and second rigid portions 80 and 90 are provided on a proximal end portion of the tube body 72 of the overtube 14. Each of the first and second rigid portions 80 and 90 includes a member having the same function as the first rigid portion 80 (see FIG. 3A) which has been described in connection with the first embodiment. Specifically, each of the mouthpieces 182 and 192 has a male configuration. In this embodiment, the main difference between the first and second rigid portions 80 and 90 resides in their sizes. A connection section 180, which is provided on the first rigid portion 80, has, preferably, a sufficiently greater size than a connection section 190 which is provided on the second rigid portion 90. Thus, a mouthpiece (not shown), which is mounted in the mouthpiece 182 on the first rigid portion 80 side, is different from a mouthpiece (not shown), which is mounted in the mouthpiece 192 on the second rigid portion 90 side. Therefore, only predetermined mouthpieces are detachably connected to the mouthpieces 182 and 192 of the first and second rigid portions 80 and 90.

As is shown in FIG. 4B, a second rigid portion 90 and a communication tube 286, which communicates with the first communication path 86, are provided on a proximal end portion of the tube body 72 of the overtube 14. The communication tube 286 has flexibility. The first mouthpiece 82 and first connection section 84, which have been described in connection with the first embodiment, are provided on the extension end portion of the communication tube 286. The second mouthpiece 92 and second connection section 94, which have been described in connection with the first embodiment, are provided on the second rigid portion 90. Thus, the mouthpiece 104 (see FIG. 3B), which is connectable to the first mouthpiece 82 on the communication tube 286 side, is different from the mouthpiece 114 (see FIG. 3B), which is connectable to the second mouthpiece 92 on the second rigid portion 90 side. Therefore, only the female first tube mouthpiece 104 (see FIG. 3B) can detachably be connected to the first mouthpiece 82 of the communication tube 286, and only the male second tube mouthpiece 114 (see FIG. 3B) can be engaged with the second mouthpiece 92 of the second rigid portion 90.

As is shown in FIG. 4C, a first communication tube 386, which communicates with the first communication path 86, and a second communication tube 396, which communicates with the second communication path 96, are provided on a proximal end portion of the tube body 72 of the overtube 14. The second communication tube 396 is formed to be longer than the first communication tube 386. The first and second communication tubes 386 and 396 have extension proximal portions at adjacent positions. The first and second communication tubes 386 and 396 have flexibility. Like the first rigid portion 80 of the first embodiment, the first mouthpiece 82 and first connection section 84 are provided at an extension end portion of the first communication tube 386. Like the second rigid portion 90 of the first embodiment, the second mouthpiece 92 and second connection section 94 are provided on the second communication tube 396. Thus, the mouthpiece 104 (see FIG. 3B), which is connectable to the first mouthpiece 82 on the first communication tube 386 side, is different from the mouthpiece 114 (see FIG. 3B), which is connectable to the second mouthpiece 92 on the second communication tube 396 side. Therefore, only the female first tube mouthpiece 104 (see FIG. 3B) can detachably be connected to the first mouthpiece 82 of the first communication tube 386, and only the male second tube mouthpiece 114 (see FIG. 3B) can detachably be connected to the second mouthpiece 92 of the second communication tube 396. In this case, it is preferable to set the total length of the first communication tube 386 and first connection tube 102 to be substantially equal to the total length of the second communication tube 396 and second connection tube 112. Thereby, if the length of the first communication 386 is greatly different from the length of the second communication tube 396, it is possible to easily discriminate between the tube 102 that is to be connected to the first communication tube 386 and the tube 112 that is to be connected to the second communication tube 396. It is also preferable to make the material of the first communication tube 386 distinguishably different from the material of the second communication tube 396, to use the same material for the first communication tube 386 and the first connection tube 102, and to use the same material for the second communication tube 396 and the second connection tube 112. In other words, it is preferable to connect the proper tubes on the basis of tactile sensation.

Although not described in connection with the first and second embodiments, it is preferable to make the colors of the first and second rigid portions 80 and 90 different. Further, it is preferable to make the shapes of the first and second rigid portions 80 and 90 different.

Next, a third embodiment of the invention is described with reference to FIG. 5. The third embodiment is a modification of the first and second embodiments. The same parts as described in the first and second embodiments or the parts with the same functions as described in the first and second embodiments are denoted by like reference numbers, and detailed description thereof is omitted.

As is shown in FIG. 5, the distal-end structure unit 42 of the endoscope 12 includes an observation optical system 420 and an illumination optical system 422. The observation optical system 420 includes an objective lens 424, a relay lens 426, a solid-state image sensing device 428 such as a CCD, and a signal line 430. The illumination optical system 422 includes an illumination lens 432 and a light guide bundle 434.

A balloon 474 for the endoscope 12, which is formed of an elastic material such as rubber and is dilatable/deflatable, is fitted over the bend portion 44 of the insertion section 22 and the distal-end structure unit 42. An overtube 14 is fitted on the insertion section 22 of the endoscope 12. A balloon 74 for the overtube 14, which is formed of an elastic material such as rubber and is dilatable/deflatable, is provided on the distal end portion of the overtube 14. A first communication path 86, which is a gas feed path, is provided in a part of the outer peripheral surface of the overtube 14. One end of the first communication path 86 communicates with the balloon 74 for the overtube 14.

An air feed path 486, which is inserted through the insertion section 22 of the endoscope 12, communicates with the balloon 474 for the endoscope 12 via an opening 486a which is formed so as to penetrate the distal-end structure unit 42 in its radial direction. A distal end portion 474a of the balloon 474 is engaged in an annular recess 42a which is formed at an edge portion of the distal-end structure unit 42. A protrusion 474b is provided at a part of the balloon 474 for the endoscope 12, which is opposed to the opening 486a. By virtue of the protrusion 474b, even if the hollow portion of the balloon 474 for the endoscope 12 is forcibly evacuated and deflated, the balloon 474 for the endoscope 12 does not completely close the opening 486a and a partially opened state of the opening 486a is maintained. Thus, when the balloon 474 for the endoscope 12 is to be deflated, the balloon 474 can be surely deflated.

A receiving surface 474c, which receives a pushing force of the distal-end tip 72a that serves as the pushing surface at the distal end of the overtube 14, is provided at the rear end of the balloon 474 for the endoscope 12. Further, a mark (not shown), which indicates a limit position for forward pushing of the overtube 14 (i.e., a position where the distal-end tip 72a of the overtube 14 contacts the receiving surface 474c of the balloon 474 for the endoscope 12) is provided on a proximal-end-side portion of the insertion section 22 of the endoscope 12.

Thus, when the overtube 14, which is fitted over the insertion section 22 of the endoscope 12, is moved forward, the limit position for forward pushing of the overtube 14 can easily be recognized on the basis of the mark provided on the insertion section 22. In addition, in the case where the overtube 14 is stayed in the body cavity and only the endoscope 12 is to be replaced, if the overtube 14 is pushed forward, the distal-end tip 72a of the overtube 14 abuts upon the receiving surface 474c of the balloon 474 for the endoscope 12, and the balloon 474 can be removed from the insertion section 22 of the endoscope 12 by the pushing operation of the overtube 14. Therefore, the endoscope 12 can easily be replaced, while the outside diameter of the overtube 14 is limited to the minimum.

Furthermore, the balloon 74 for the overtube 14 is dilated and the outer periphery of the balloon 74 is put in close contact with the intestinal wall and is fixed. Thereby, the replacement work of the endoscope 12 is facilitated, and this embodiment can be applied to an endoscope system wherein the balloon-equipped endoscope 12 is used at the time of insertion and a large-channel endoscope, which is dedicated to surgery, is used at the time of surgery. Hence, efficiency both in insertion and surgery can be realized. Moreover, if a surgical instrument having an electromagnet function is inserted in the channel of the large-channel endoscope or in the hollow portion of the balloon-equipped overtube, the recovery of a capsule endoscope in the body cavity becomes easier.

As has been described above, the present embodiments can provide an overtube for an endoscope 12 and an endoscope system 10, which can easily determine non-connectability between erroneously combined connection members, and can easily determine mutually connectable members and can connect these members.

## Claims

1. An overtube (14) for an endoscope (12), **characterized by** comprising:
a tube body (72) having a distal end portion and a proximal end portion and including a hollow portion in which an insertion section (22) of an endoscope (12) is inserted;
a balloon (74) which is dilatably and deflatably provided on an outer peripheral surface of the tube body;
a first communication path (86) which extends toward the proximal end portion of the tube body in a state in which the first communication path communicates with the balloon;
a second communication path (96) which extends from the hollow portion toward the proximal end portion of the tube body in a state in which the second communication path communicates with the hollow portion;
a first mouthpiece (82) which projects from the outer peripheral surface of the tube body and includes a path communicating with the first communication path;
a second mouthpiece (92) which projects from the outer peripheral surface of the tube body and includes a path communicating with the second communication path;
a first connection section (84) which is provided on the first mouthpiece, the first connection section being connected to a first feed portion (106) of a fluid feed mechanism and being prevented from being connected to a second feed portion (116) of the fluid feed mechanism; and
a second connection section (94) which is provided on the second mouthpiece, the second connection section being connected to the second feed portion of the fluid feed mechanism and being prevented from being connected to the first feed portion of the fluid feed mechanism.

2. The overtube (14) for an endoscope (12) according to claim 1, **characterized in that** the first connection section (84) has one of a male configuration and a female configuration, and the second connection section (94) has a female configuration when the first connection section has a male configuration, and has a male configuration when the first connection section has a female configuration.

3. The overtube (14) for an endoscope (12) according to claim 1 or 2, **characterized in that** the first mouthpiece (82) and the second mouthpiece (92) are provided on mutually opposed sides with respect to a longitudinal axis of the tube body (72).

4. The overtube (14) for an endoscope (12) according to claim 1 or 2, **characterized in that** the first mouthpiece (82) and the second mouthpiece (92) project from mutually adjacent positions on the tube body (72).

5. The overtube (14) for an endoscope (12) according to any one of claims 1 to 4, **characterized in that** at least one of the first mouthpiece (82) and the second mouthpiece (92) is connected via a flexible tube (286; 386, 396) to the tube body (72).

6. The overtube (14) for an endoscope (12) according to claim 5, **characterized in that** in a case where the flexible tubes (386, 396) are provided between the tube body (72) and the first mouthpiece (82) and the second mouthpiece (92), the flexible tubes have different lengths (L₁, L₂).

7. The overtube (14) for an endoscope (12) according to any one of claims 1 to 6, **characterized in that** the first connection section (84) and the second connection section (94) are different colors, by which the first connection section and the second connection section are recognized as being different.

8. An endoscope system (10) **characterized by** comprising:
an endoscope (12), the endoscope including:
an insertion section (22) having a distal end portion and a proximal end portion and including a bend portion (44) which is bendable; and
an operation section (24) which is connected to the insertion section (22) and is capable of performing an operation of bending the bend portion; and
an overtube (14), the overtube including:
a tube body (72) having a distal end portion and a proximal end portion and including a hollow portion in which the insertion section is detachably inserted such that the tube body covers a part of the insertion section;
a balloon (74) provided on an outer peripheral surface of the tube body;
a first communication path (86) which has one end communicating with the balloon and the other end extending toward the proximal end portion of the tube body along the outer peripheral surface of the tube body;
a second communication path (96) which has one end communicating with the hollow portion of the tube body and the other end extending toward the proximal end portion of the tube body;
a first connection section (84) which is provided on an outside of the tube body, includes a first mouthpiece (82) communicating with the other end of the first communication path, is connectable to a first fluid feed mechanism (102) which feeds and drains a fluid to and from the first communication path, and is prevented from being connected to a second fluid feed mechanism (112) which feeds and drains a fluid to and from the second communication path; and
a second connection section (94) which is provided on the outside of the tube body, formed in a shape different from a shape of the first connection section, includes a second mouthpiece (92) communicating with the other end of the second communication path, is connectable to the second fluid feed mechanism which feeds and drains a fluid to and from the second communication path, and is prevented from being connected to the first fluid feed mechanism.

9. The endoscope system (10) according to claim 8, **characterized in that** the first mouthpiece (82) and the second mouthpiece (92) are provided on mutually opposed sides with respect to a longitudinal axis of the tube body (72).

10. The endoscope system (10) according to claim 8, **characterized in that** the first mouthpiece (82) and the second mouthpiece (92) project from mutually adjacent positions on the tube body (72).

11. The endoscope system (10) according to any one of claims 8 to 10, **characterized in that** at least one of the first mouthpiece (82) and the second mouthpiece (92) is connected via a flexible tube (286; 386, 396) to the tube body (72).

12. The endoscope system (10) according to claim 11, **characterized in that** in a case where the flexible tubes (386, 396) are provided between the tube body (72) and the first mouthpiece (82) and the second mouthpiece (92), the flexible tubes have different lengths (L₁, L₂).

13. The endoscope system (10) according to any one of claims 8 to 12, **characterized in that** the first connection section (84) and the second connection section (94) are different colors, by which the first connection section and the second connection section are recognized as being different.

14. The endoscope system (10) according to any one of claims 8 to 13, **characterized in that** the first connection section (84) has one of a male configuration and a female configuration such that the first connection section is connected to the first fluid feed mechanism (102), and
the second connection section (94) is connected to the second fluid feed mechanism (112), has a female configuration when the first connection section has a male configuration, and has a male configuration when the first connection section has a female configuration.

15. The endoscope system (10) according to any one of claims 8 to 14, **characterized in that** a balloon (474), which is different from said balloon (74) of the overtube (14), is provided at the distal end portion of the insertion section (22).
